(19) Europäisches Patentamt
European Patent Office
Office européen des brevets

(11) Veröffentlichungsnummer: **0 419 988 A1**

# (12) EUROPÄISCHE PATENTANMELDUNG

(21) Anmeldenummer: **90117836.8**

(22) Anmeldetag: **17.09.90**

(51) Int. Cl.$^5$: **C12P 41/00**, C12P 7/62, C12P 17/06

(30) Priorität: **25.09.89 LU 87593**

(43) Veröffentlichungstag der Anmeldung: **03.04.91 Patentblatt 91/14**

(84) Benannte Vertragsstaaten: **AT BE CH DE ES FR GB GR IT LI LU NL SE**

(71) Anmelder: **Europäische Wirtschaftsgemeinschaft Bâtiment Jean Monnet**
**Plateau du Kirchberg**
**L-2920 Luxemburg(LU)**

(72) Erfinder: **Schapöhler, Stefan**
**Wilhelm-Busch-Strasse 8-10**
**W-3000 Hannover 1(DE)**
Erfinder: **Schügerl, Karl, Prof. Dr.**
**Arnumer Kirchstrasse 31**
**W-3005 Hemmingen 4(DE)**
Erfinder: **Scheper, Thcmas, Dr.**
**Däumlingweg 6**
**W-3000 Hannover 1(DE)**

(74) Vertreter: **Weinmiller, Jürgen**
**Lennéstrasse 9 Postfach 24**
**W-8133 Feldafing(DE)**

## (54) Enantioselektive enzymatische Synthese von S(-)- und R(+)-Estern des 4-Hydroxy-cyclopenten-1-ons und seines 2',2'-Dimethylpropan-1',3'-diol-ketals.

(57) Die S(-)- und R(+)-Ester des 4-Hydroxy-cyclopenten-1-ons und seines 2',2'-Dimethylpropan-1',3'-diol-ketals werden hergestellt durch direkte enantioselektive enzymatische Synthese aus den entsprechenden 4-Hydroxy-verbindungen durch Umsetzung mit einem Ester als Acyldonor in Gegenwart eines Enzyms.

Die dabei erhaltenen Ester stellen wertvolle Zwischenprodukte für stereospezifische Syntheseverfahren, insbesondere für die stereoselektive Synthese chemischer Verbindungen, insbesondere stabiler, lagerfähiger, chiraler Prostaglandinderivate, speziell des lagerfähigen chiralen Prostaglandinsynthons, dar.

EP 0 419 988 A1

# ENANTIOSELEKTIVE ENZYMATISCHE SYNTHESE VON S(-)- UND R(+)-ESTERN DES 4-HYDROXY-CYCLOPENTEN-1-ONS UND SEINES 2′,2′-DIMETHYLPROPAN-1′,3′-DIOL-KETALS

Die Erfindung betrifft die enantioselektive enzymatische Synthese von S(-)- und R(+)-Estern des 4-Hydroxy-cyclopenten-1-ons und seines 2′,2′-Dimethylpropan-1′,3′-diol-ketals, sie betrifft insbesondere ein Verfahren zur enantioselektiven Herstellung der genannten S(-)- und R(+)-Ester der nachstehend angegebenen allgemeinen Formel (Ia) bzw. (Ib)

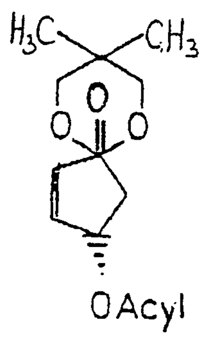

(Ia)

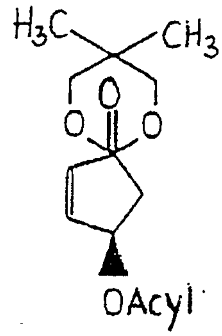

(Ib)

die wertvolle Zwischenprodukte für die gezielte, d.h. stereo-bzw. enantioselektive Synthese chemischer Verbindungen, insbesondere stabiler, lagerfähiger chiraler Prostaglandinderivate, darstellen.

Aus der DE-OS 37 24 721 ist es bereits bekannt, aus racemischem Ketalacetat durch hydrolytische Esterspaltung in Gegenwart eines Enzyms, d.h. durch enzymatische Hydrolyse, den entsprechenden S(-)-Alkohol herzustellen unter Zurückbleiben des R(+)-Esters. Der in dem Reaktionsgemisch (pH 7) enthaltene (S(-)-Alkohol ist jedoch in dem wäßrigen Reaktionssystem instabil und seine Weiterverarbeitung zum S(-)-Ester durch chemische Veresterung ist technisch außerordentlich kompliziert, wobei das gewünschte Endprodukt nur in verhältnismäßig geringer Ausbeute erhalten wird.

Aufgabe der Erfindung war es daher, einen Weg zur selektiven Herstellung der S(-)- und R(+)-Ester des 4-Hydroxy-2-cyclopenten-1-ons und seines 2′,2′-Dimethylpropan-1′,3′-diol-ketals zu finden, der technisch einfach ist und die gewünschten Endprodukte in hoher Reinheit und Ausbeute liefert.

Es wurde nun gefunden, daß diese Aufgabe erfindungsgemäß dadurch gelöst werden kann, daß in Umkehrung zu der bereits bekannten enantioselektiven enzymatischen Spaltung des racemischen Ketalacetats in einem auf pH 7 abgepufferten wäßrigen Medium die S(-)- und R(+)-Ester des 4-Hydroxy-2-cyclopenten-1-ons und seines 2′,2′-Dimethylpropan-1′,3′-diol-ketals durch gezielte, d.h. stereo- bzw. enantioselektive enzymatische Synthese aus der entsprechenden racemischen Hydroxylverbindung und einem Säureester als Acyldonor in Gegenwart eines Enzyms in einem organischen Lösungsmittel hergestellt werden.

Gegenstand der Erfindung ist ein Verfahren zur enantioselektiven Herstellung von S(-)- und R(+)-Estern des 4-Hydroxy-cyclopenten-1-ons und seines 2′,2′-Dimethylpropan-1′,3′-diol-ketals der allgemeinen Formel (Ia) bzw. (Ib)

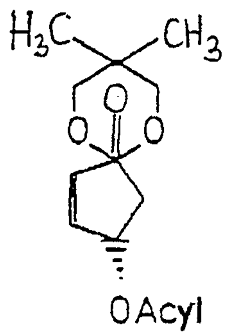

(Ia)

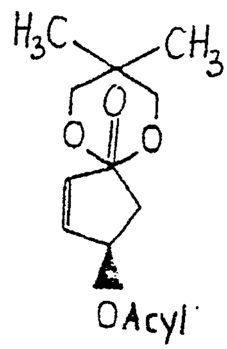

(Ib)

das dadurch gekennzeichnet ist, daß man

a) ein racemisches Gemisch des 4-Hydroxy-2-cyclopenten-1-ons oder seines 2',2'-Dimethylpropan-1',3'-diol-ketals der Formel (II) in Gegenwart eines Enzyms mit einem Ester der allgemeinen Formel (III) als Acyldonor in organischer Phase, vorzugsweise in wasserfreier organischer Phase, bei Raumtemperatur oder erhöhter Temperatur umsetzt unter Bildung des S(-)-Esters (Ia) und unter Zurückbleiben des R(+)-Alkohols der Formel (IIb) entsprechend der Reaktionsgleichung:

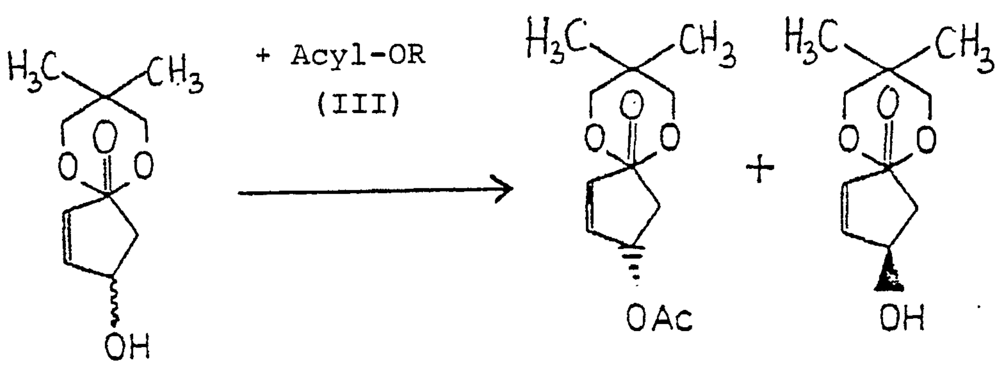


(II) (Ia) (IIb)

+ R - OH
(IV)

worin Acyl einen beliebigen Säurerest, vorzugsweise den Rest einer organischen $C_1$-$C_{22}$-Carbonsäure, und R eine gegebenenfalls substituierte, unverzweigte oder verzweigte Alkyl-, Alkenyl- oder Alkinylgruppe mit 1 bis 22, vorzugsweise 1 bis 12, insbesondere 1 bis 6 Kohlenstoffatomen, eine gegebenenfalls substituierte Arylgruppe mit 6 bis 12, vorzugsweise 6 bis 10 Kohlenstoffatomen, oder eine gegebenenfalls substituierte heterocyclische Gruppe mit 5 bis 10, vorzugsweise 5 bis 7 Ringatomen, die mindestens ein Stickstoff-, Sauerstoff- und/oder Schwefelatom als Heteroatom(e) enthält, bedeuten,

b) das in der Stufe (a) erhaltene Produktgemisch auf chromatographischem oder extraktivem Wege auftrennt in den S(-)-Ester der Formel (Ia), der als erstes Endprodukt gewonnen wird, und in den R(+)-Alkohol der Formel (IIb), der

c) durch chemische oder enzymatische Veresterung mit dem Ester der Formel (III) in den R(+)-Ester der Formel (Ib) überführt wird, der seinerseits auf chromatographischem oder extraktivem Wege in reiner Form als zweites Endprodukt gewonnen wird, entsprechend der Reaktionsgleichung:

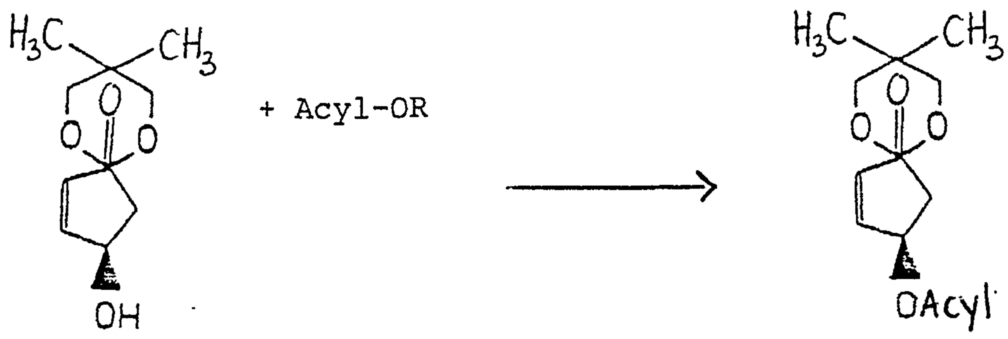


(IIb) (III) (Ib)

+ R - OH

(IV)

Nach dem erfindungsgemäßen Verfahren ist es auf technisch einfache und wirtschaftliche Weise möglich, durch direkte enantioselektive enzymatische Synthese unter Umgehung der instabilen S(-)-Hydroxyverbindung die S(-)- und R(+)-Ester des 4-Hydroxy-2-cyclopenten-1-ons und seines 2′,2′-Dimethylpropan-1′,3′-diol-ketals herzustellen, die wertvolle Zwischenprodukte für die Synthese eines stabilen, lagerfähigen chiralen Prostaglandinsynthons darstellen. Insbesondere das 4-Acetoxy-cyclopentenon bzw. sein Ketal sind Schlüsselsubstanzen in der Synthese cyclopentanoider Naturstoffe (vgl. DE-OS 37 24 721, R. Noyori et al, "Angewandte Chemie", 96 , 854 (1984), und E. Winterfeldt et al, "Angewandte Chemie", 94 , 496 (1982)).

Bei der Durchführung des erfindungsgemäßen Verfahrens werden die gewünschten Endprodukte in stabiler Form und außerordentlich hoher Reinheit und Ausbeute erhalten. Darüber hinaus bietet das erfindungsgemäße Verfahren gegenüber dem aus der DE-OS 37 24 721 bekannten Verfahren die folgenden technischen Vorteile: Neben der geringeren Anzahl der Synthesestufen kommt es zu einer deutlichen Verminderung des Lösungsmittelbedarfs; die Reaktion läuft in sehr geringen Volumina ab; ein Einsatz giftiger bzw. cancerogener Extraktionslösungsmittel entfällt, da direkt aufgearbeitet werden kann, was auch mit Energieersparnis verbunden ist; das Enzym läßt sich leicht abtrennen (beispielsweise durch Abfiltrieren) und es ist keine Immobilisierung erforderlich; ein kontinuierlicher Einsatz mit einer Enzymkartusche (Festbett) ist ohne weiteres möglich; und ein Einsatz von Lösevermittlern entfällt.

Bei der Durchführung des erfindungsgemäßen Verfahrens wird als Acyldonor vorzugsweise ein Ester der allgemeinen Formel verwendet

$$R_1 - \overset{O}{\overset{\|}{C}} - OR_2 \qquad V$$

worin $R_1$ und $R_2$, die gleich oder verschieden sein können, jeweils die vorstehend für R angegebenen Bedeutungen haben können.

Ganz besonders bevorzugt ist die Verwendung von Essigsäureethylester (Ethylacetat) und eines Glycerintriesters einer organischen Säure mit 1 bis 22 Kohlenstoffatomen, insbesondere von Triacetin, Tributyrin und dgl., als Acyldonor, wobei sich letztere durch ihre Ungiftigkeit und ihre hohe Reaktionsgeschwindigkeit auszeichnen.

Bei der Durchführung des erfindungsgemäßen Verfahrens ist der extraktiven Aufarbeitung die übliche Flash-Säulenchromatogrphie vorzuziehen (vgl. Still et al, "J. Org. Chem.", 43 , 2 923 (1978)), wie sie auch in den weiter unten folgenden Ausführungsbeispielen angewendet wurde. Bei der Durchführung der Stufe (c) des erfindungsgemäßen Verfahrens ist die enzymatische Veresterung der chemischen Standardveresterung (mit einem Säurechlorid/Säureanhydrid und Pyridin und einem Alkohol) vorzuziehen. Die Durchführung dieses Verfahrens entspricht der Durchführung der enantioselektiven Veresterung. Die direkte enantioselektive enzymatische Veresterung gemäß der vorliegenden Erfindung ist besonders vorteilhaft bei der ketalisierten Verbindung, wobei man mit dem 2′,2′-Dimethylpropan-1′,3′-diolketal (= Ketalalkohol) eine vollständige Trennung der Enantiomeren erzielen kann, wobei durch anschließende Deketalisierung leicht die

enantiomerenreinen Ester des nicht-ketalisierten 4-Hydroxycyclopentenons (= Ketoalkohol) erhalten werden (beispielsweise unter katalytischer Essigsäure- bzw. Ameisensäurezugabe bei Raumtemperatur durch 1-stündiges Schütteln oder durch 48-stündiges Stehenlassen auf Merck-Kieselgel Nr. 9385).

Während im Falle des Ketosubstrats der Formel (II) in der Stufe (a) des erfindungsgemäßen Verfahrens als Enzym vorzugsweise Schweineleberesterase verwendet wird, wird im Falle des Ketalsubstrats der Formel (II) als Enzym vorzugsweise Lipase, insbesondere eine Lipase verwendet, die aus Pseudomonas fluorescens (Amano Lipase P, Charge-Nr. LPL 05518) gewonnen worden ist.

Eine 60 %ige enantioselektive enzymatische Veresterung beim Ketosubstrat wird erfindungsgemäß erreicht mit Pseudomonas fluorescens-Lipase (z.B. Amano Lipase P, Charge Nr. LPL 05518 oder Röhm, EL 220-88). Bei der ketalisierten Verbindung kann mit den folgenden Lipasen eine vollständige Enantiomerentrennung erreicht werden: Lipasen aus Pseudomonas fluorescens (z.B. Amano Lipase P, Charge Nr. LPL 05518 oder Röhm, EL 220-88), Candida cylindracea (Hersteller u.a. Amano Lipase AY, Charge Nr. LAY MO 3517 oder Sigma, Kat.-Nr. L-1754, Lot-Nr. 34F-0621), Porcine pancreas (Hersteller u.a. Röhm, Charge-Nr. 7023 C, Sigma, Kat.-Nr. L-3126, Lot-Nr. 74F-0470) und Mucor miehei (Gist-Brocades, Charge-Nr. 0282).

Die Enzyme werden vorzugsweise im Überschuß eingesetzt. Massenverhältnisse von Enzym/Alkohol von 0,5:1 bis 10:1 haben sich als vorteilhaft erwiesen, je nach Aktivität (U) des Enzyms (Bezugsreaktion: Hydrolyse eines Triglycerids bzw. Lösungsmittelesters, U = $\mu$Mol Fettsäureäquivalent pro Minute bei konstantem pH-Wert in wäßriger Emulsion, auch entsprechend den Angaben der Enzymhersteller). Die immobilisierte Pseudomonas-Lipase von Röhm weist beispielsweise eine 6-fach geringere spezifische Aktivität auf, muß also entsprechend höher dosiert werden.

Bei der Durchführung des erfindungsgemäßen Verfahrens wird in der Stufe (a) als organisches Lösungsmittel vorzugsweise ein Kohlenwasserstoff, wie n-Heptan, i-Octan etc., oder ein Ester, insbesondere Cyclohexylacetat, speziell der als Acyldonor eingesetzte Ester, insbesondere Tributyrin, verwendet.

Die in der Stufe (a) des erfindungsgemäßen Verfahrens durchgeführte Umesterung kann bei Raumtemperatur oder erhöhter Temperatur, vorzugsweise bei einer Temperatur in dem Bereich von 40 bis 75° C, insbesondere 58 bis 62° C, speziell bei 60° C, durchgeführt werden.

Das organische Lösungsmittel und der Alkohol der Formel (II) werden in der Stufe (a) des erfindungsgemäßen Verfahrens vorzugsweise in einem Massenverhältnis von 4:1 bis 100:1, insbesondere von 5:1 bis 10:1, eingesetzt, wobei das Verhältnis von Lösungsmittel zu Alkohol maßgeblich von der Löslichkeit des verwendeten Alkohol mitbestimmt wird.

Das Enzym und der Alkohol der Formel (II) werden in der Stufe (a) des erfindungsgemäßen Verfahrens vorzugsweise in einem Massenverhältnis von 0,5:1 bis 10:1, speziell 0,5:1 bis 1,5:1, insbesondere 0,7:1 bis 0,9:1, eingesetzt.

Gemäß einer besonders bevorzugten Ausgestaltung des erfindungsgemäßen Verfahrens kann anstelle der Veresterung des R(+)-Alkohols der Formel (IIb) in der Stufe (c) eine Racemisierung des Alkohols und Rückführung desselben in die Stufe (a) durchgeführt werden, wodurch eine vollständige enantioselektive Veresterung der racemischen Hydroxyverbindung der Formel (II) zum S(-)-Ester der Formel (Ia) erzielt werden kann.

Der Reaktionsverlauf und das Enantiomerenverhältnis können durch Einsatz von Hochdruckflüssigkeitschromatographie (HPLC, mit der chiralen HPLC-Säule Daicel OA, Eluierungsmittel n-Hexan/Isopropanol 10:1) oder Kapillargaschromatographie (Säule Lipodex® A von Macherey & Nagel) bestimmt werden. Die Einzelheiten dieser Verfahren entsprechen dem allgemeinen Laborstandard (unter Berücksichtigung der Manuale der Hersteller). Die Bestätigung des Enantiomerenverhältnisses und der absoluten Konfiguration kann schließlich standardmäßig durch H-NMR der Mosher-Ester erfolgen (vgl. J.A. Dale et al, "JACS", 95, 512-519 (1973), und DE-OS 37 24 721).

Das erfindungsgemäß verwendete Enzym kann in freier Form, d.h. gelöst oder suspendiert in dem organischen Reaktionsmedium, oder in immobilisierter Form, beispielsweise in einer in einem Polymersubstrat eingeschlossenen Form, eingesetzt werden.

Die vorstehend angegebenen Formeln Ia, Ib, II und IIb stellen Kombinationsformeln dar, in denen die Strukturformeln des 4-Hydroxy-cyclopenten-1-ons und seiner Esterderivate einerseits sowie seines 2′,2′-Dimethylpropan-1′,3′-diol-ketals und seiner Esterderivate andererseits zur Vereinfachung der Reaktionsgleichungen zusammengefaßt sind. So stellt beispielsweise die Formel (II) eine Kombination aus den Formeln (II′) und (II″) dar:

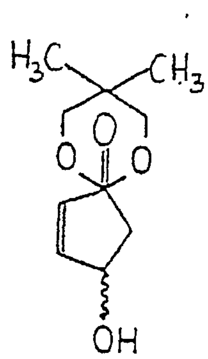

=

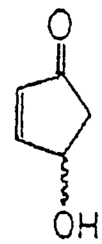

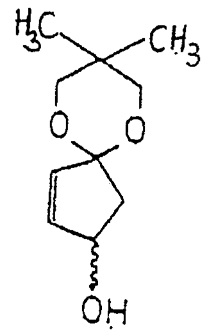

(II) (II') (II")

Unter Berücksichtigung der vorstehenden Definition kann der Ablauf des erfindungsgemäßen Verfahrens bei Verwendung von Essigsäureethylester als Acyldonor für die beiden Ausgangsverbindungen 4-Hydroxy-cyclopenten-1-on (A) und seines 2′,2′-Dimethylpropan-1′,3′-diol-ketals (B) schematisch wie folgt dargestellt werden (Ac = Acetyl):

A)

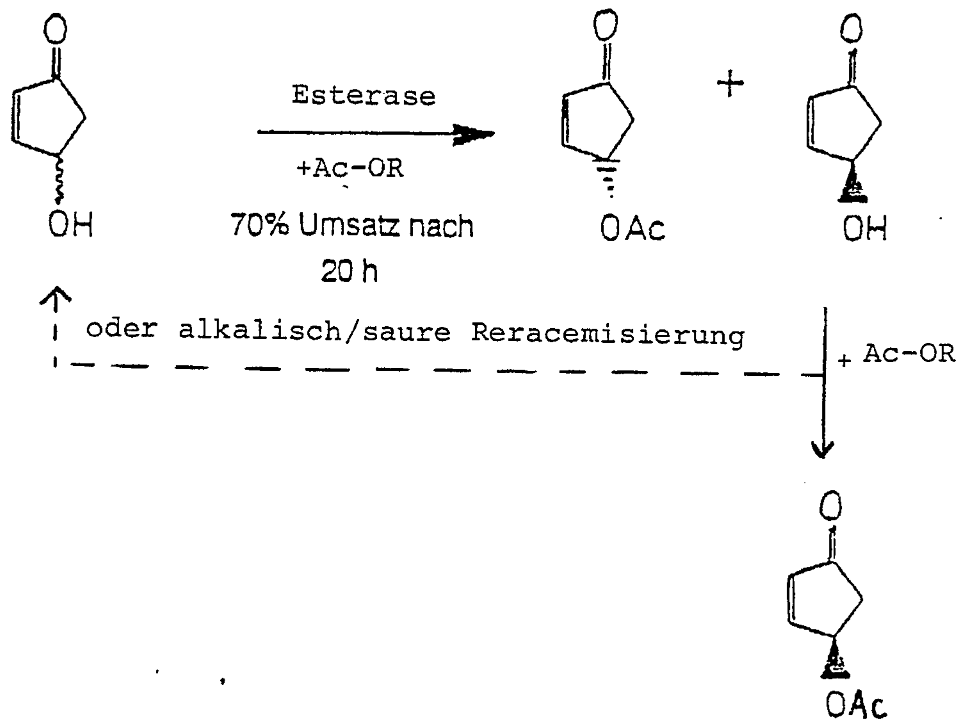
Esterase
+Ac-OR
70% Umsatz nach
20 h
oder alkalisch/saure Reracemisierung
+ Ac-OR

B)

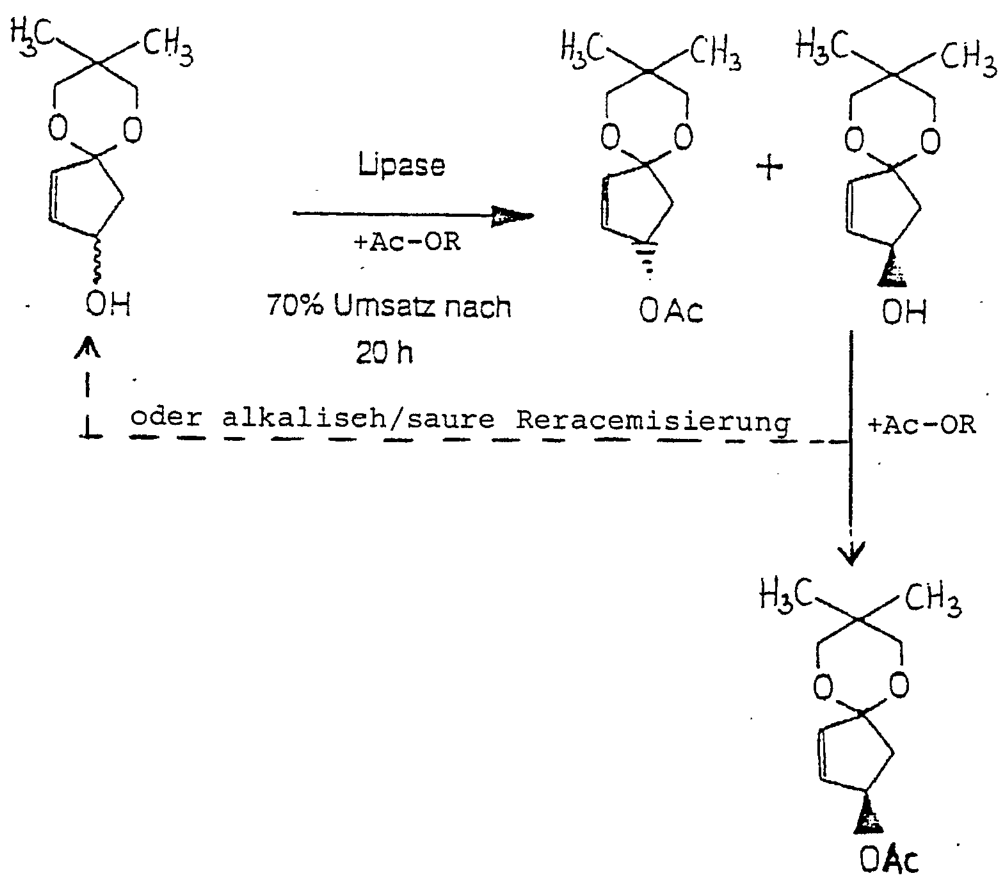
Lipase
+Ac-OR
70% Umsatz nach
20 h
oder alkaliseh/saure Reracemisierung
+Ac-OR

Die geeigneten Reaktionsparameter für die Acetylierung gemäß dem erfindungsgemäßen Verfahren hängen von den sich ändernden Randbedingungen, beispielsweise von den Massenverhältnissen oder der jeweiligen Aktivität des jeweils verwendeten Enzyms, von der Wahl des gewünschten Säurerestes (z.B. Essigsäure-, Buttersäure-, Ölsäurerest) und dgl. ab. Die optimalen Parameter für die Acetylierung nach dem erfindungsgemäßen Verfahren sind insbesondere folgende:

Enzym: Schweineleberesterase bzw. Lipase aus Pseudomonas fluorescens (Amano Lipase P, Charge-Nr. LPL 05518)

Lösungsmittel (LM): Triacetin (Glycerintriacetat, vollkommen umweltneutral, da ein Naturstoff)

Massenverhältnis getrockneter Ester (LM)/Ketalalkohol: 6:1

Massenverhältnis Enzym/-Ketalalkohol 1:1

Reaktionstemperatur: 40 °C ± 1 °C

Wassergehalt des Enzyms: 3,17 % (lyophilisiertes Enzym, direkt vom Hersteller zur Reaktion eingesetzt)

Die Erfindung wird durch die folgenden Beispiele näher erläutert, ohne jedoch darauf beschränkt zu sein.

Beispiel 1

In einem 10 ml Rundkolben im Ölbad bei 40 °C auf einem Magnetrührer wurden 521,3 mg (= 2,83 mmol) Ketalalkohol, 511,3 mg Amano Lipase P (Chargen-Nr. LPL 05518) in 3 ml (= 3,1 g = 9 mmol) Tributyrin zur Reaktion gebracht.

Nach 45 h wurde die Reaktion abgebrochen, das Enzym abfiltriert, mit Aceton gewaschen und das gesamte Filtrat auf einer 50 g Kieselsäule (Merck Flashkieselgel 60, Nr. 9385) mit 2:1 Petroläther/Äther als Laufmittel aufgetrennt.

Es wurden 240,1 mg (= 0,98 mmol) S(-)Ketalbutyrat (Enantiomerenreinheit> 95 %ee, lt. HPLC) und 281,2 mg (= 1,53 mmol) R(+)Ketallakohol (> 95 %ee, lt. HPLC) erhalten.

Beispiel 2

In einem 10 ml Rundkolben im Ölbad bei 60 °C auf einem Magnetrührer wurden 2 ml getrockneter Essigsäurecyclohexylester (13,6 mmol), 250 mg Amano Lipase P (wie in Beispiel 1) und 308,0 mg (= 1,68 mmol) Ketalalkohol zur Reaktion gebracht.

Nach 20 h wurde die Reaktion abgebrochen, das Enzym abfiltriert, mit Aceton gewaschen und das Filtrat auf einer 50 g Kieselsäule (Merck, Nr. 9385) mit einem Petroläther/Äther (1:1)-Gemisch aufgetrennt. Ausbeute: 140,2 mg (0,76 mmol) R(+)Ketalalkohol (> 95 %ee lt. HPLC und H-NMR des Mosher-Esters) und 203,4 mg (= 0,83 mmol) S(-)Ketalbutyrat (> 95 %ee lt. HPLC).

## Ansprüche

1. Verfahren zur enantioselektiven Herstellung von S(-)- und R(+)Estern des 4-Hydroxy-cyclopenten-1-ons und seines 2′,2′-Dimethylpropan-1′,3′-diol-ketals der allgemeinen Formel (Ia) bzw. (Ib)

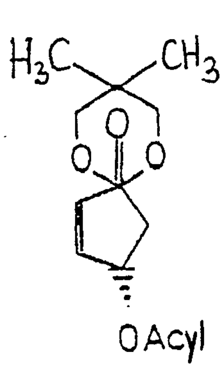

(Ia)

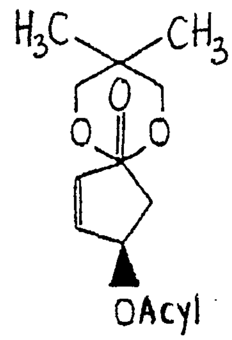

(Ib)

dadurch **gekennzeichnet,** daß

a) ein racemisches Gemisch des 4-Hydroxy-2-cyclopenten-1-ons oder seines 2′,2′-Dimethylpropan-1′,3′-diol-ketals der nachstehenden Formel (II) in Gegenwart eines Enzyms mit einem Ester der nachstehenden allgemeinen Formel (III) als Acyldonor in organischer Phase umgesetzt wird unter Bildung des entsprechenden S(-)Esters der allgemeinen Formel (Ia) und unter Zurückbleiben des R(+)-Alkohols der Formel (IIb) sowie unter Bildung des Alkohols der allgemeinen Formel (IV)

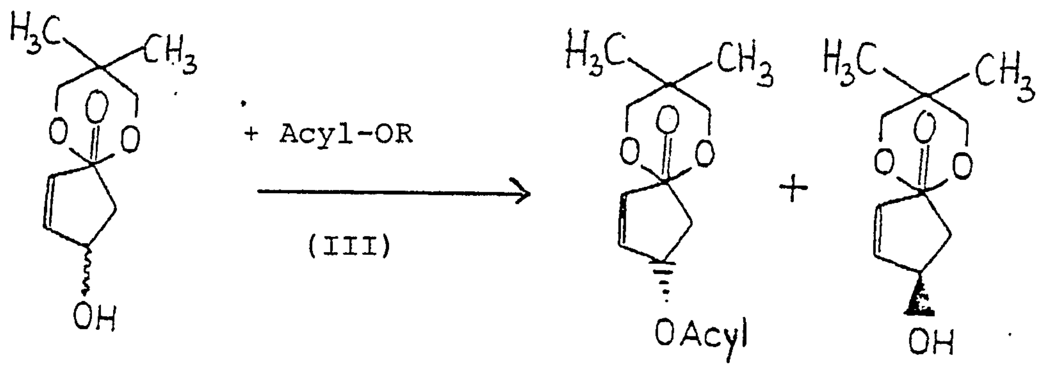


(II) (Ia) (IIb)

+ R - OH

(IV)

worin Acyl einen beliebigen Säurerest, vorzugsweise den Rest einer organischen $C_1$-$C_{22}$-Carbonsäure, und R eine gegebenenfalls substituierte, unverzweigte oder verzweigte Alkyl-, Alkenyl- oder Alkinylgruppe mit 1 bis 22, vorzugsweise 1 bis 12, insbesondere 1 bis 6 Kohlenstoffatomen, eine gegebenenfalls substituierte Arylgruppe mit 6 bis 12, vorzugsweise 6 bis 10 Kohlenstoffatomen oder eine gegebenenfalls substituierte heterocyclische Gruppe mit 5 bis 10, vorzugsweise 5 bis 7 Ringatomen, die mindestens ein Stickstoff-, Sauerstoff- und/oder Schwefelatom als Heteroatom(e) enthält, bedeuten,

b) das in der Stufe (a) erhaltene Produktgemisch auf chromatographischem oder extraktivem Wege aufgetrennt wird in den S(-)-Ester der Formel (Ia), der als erstes Endprodukt gewonnen wird, und in den R(+)-Alkohol der Formel (IIb), der

c) durch chemische oder enzymatische Veresterung mit dem Ester der Formel (III) in den R(+)-Ester der Formel (Ib) überführt wird, der seinerseits auf chromatographischem oder extraktivem Wege in reiner Form als zweites Endprodukt gewonnen wird, entsprechend der Reaktionsgleichung:

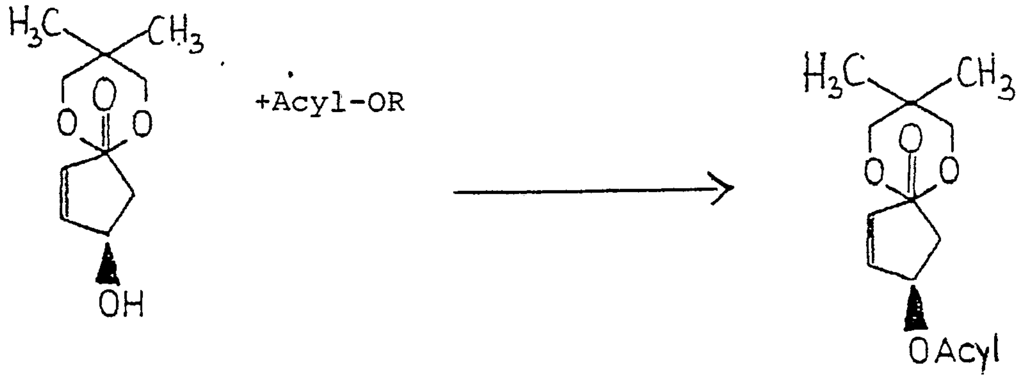


(IIb) (III) (Ib)

+ R - OH

(IV)

2. Verfahren nach Anspruch 1, dadurch gekennzeichnet, daß als Acyldonor ein Ester der allgemeinen Formel verwendet wird:

$$R_1 - \overset{O}{\overset{\|}{C}} - OR_2 \qquad (V)$$

worin $R_1$ und $R_2$, die gleich oder verschieden sein können, jeweils die für R in Anspruch 1 angegebenen Bedeutungen haben.

3. Verfahren nach Anspruch 1 oder 2, dadurch gekennzeichnet, daß als Acyldonor Essigsäureethylester (Ethylacetat) oder ein Glycerintriester einer organischen Säure mit bis zu 22 Kohlenstoffatomen, insbesondere Triacetin oder Tributyrin, verwendet wird.

4. Verfahren nach einem der Ansprüche 1 bis 3, dadurch gekennzeichnet, daß im Falle des Ketosubstrats der Formel (II) als Enzym Schweineleberesterase verwendet wird.

5. Verfahren nach einem der Ansprüche 1 bis 3, dadurch gekennzeichnet, daß im Falle des Ketalsubstrats der Formel (II) als Enzym Lipase aus Pseudomonas fluorescens verwendet wird.

6. Verfahren nach einem der Ansprüche 1 bis 5, dadurch gekennzeichnet, daß in der Stufe (a) als organisches Lösungsmittel ein Kohlenwasserstoff und/oder ein Ester, vorzugsweise der als Acyldonor eingesetzte Ester, verwendet wird.

7. Verfahren nach einem der Ansprüche 1 bis 6, dadurch gekennzeichnet, daß die Umesterung in der Stufe (a) bei Raumtemperatur oder erhöhter Temperatur, vorzugsweise bei einer Temperatur in dem Bereich von 40 bis 75 °C, insbesondere 58 bis 62 °C, speziell bei 60 °C, durchgeführt wird.

8. Verfahren nach einem der Ansprüche 1 bis 7, dadurch gekennzeichnet, daß in der Stufe (a) das organische Lösungsmittel und der Alkohol der Formel (III) in einem Massenverhältnis von 4:1 bis 100:1, vorzugsweise 5:1 bis 10:1, eingesetzt werden.

9. Verfahren nach einem der Ansprüche 1 bis 8, dadurch gekennzeichnet, daß in der Stufe (a) das Enzym und der Alkohol der Formel (III) in einem Massenverhältnis von 0,5:1 bis 10:1, speziell 0,5:1 bis 1,5:1, insbesondere 0,7:1 bis 0,9:1, eingesetzt werden.

10. Verfahren nach einem der Ansprüche 1 bis 9, dadurch gekennzeichnet, daß anstelle der Veresterung des R(+)-Alkohols der Formel (IIb) in der Stufe (c) eine Racemisierung des R(+)-Alkohols und eine Rückführung desselben in die Stufe (a) durchgeführt werden zur vollständigen enantioselektiven Überführung der racemischen Verbindung der Formel (II) in den S(-)-Ester der Formel (Ia).

Europäisches Patentamt

# EUROPÄISCHER RECHERCHENBERICHT

Nummer der Anmeldung

EP 90 11 7836

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | CHEMICAL ABSTRACTS, Band 99, Nr. 25, 19. Dezember 1983, Seite 531, Zusammenfassung Nr. 211169u, Columbus, Ohio, US; & JP-A-58 138 386 (SUMITOMO CHEMICAL CO., LTD) 17-08-1983 * Zusammenfassung * --- | 1 | C 12 P 41/00<br>C 12 P 7/62<br>C 12 P 17/06 |
| Y,D | DE-A-3 724 721 (HOECHST) * Patentansprüche * --- | 1 | |
| Y | EP-A-0 127 386 (SUMITOMO) * Patentansprüche * --- | 1 | |
| Y | CHEMICAL ABSTRACTS, Band 111, Nr. 15, 9. Oktober 1989, Seite 625, Zusammenfassung Nr. 132612w, Columbus, Ohio, US; & JP-A-01 20 091 (SUMITOMO CHEMICAL CO., LTD) 24-01-1989 * Zusammenfassung * --- | 1 | |
| Y | CHEMICAL ABSTRACTS, Band 109, Nr. 19, 7. November 1988, Seite 597, Zusammenfassung Nr. 168996x, Columbus, Ohio, US; & JP-A-63 00 292 (SUMITOMO CHEMICAL CO., LTD) 05-01-1988 * Zusammenfassung * ----- | 1 | RECHERCHIERTE SACHGEBIETE (Int. Cl.5)<br>C 12 P |

Der vorliegende Recherchenbericht wurde für alle Patentansprüche erstellt

| Recherchenort | Abschlußdatum der Recherche | Prüfer |
|---|---|---|
| DEN HAAG | 12-12-1990 | DELANGHE L.L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument